(19) Europäisches Patentamt
European Patent Office
Office européen des brevets

(11) Veröffentlichungsnummer: **0 147 706**
**B1**

(12) # EUROPÄISCHE PATENTSCHRIFT

(45) Veröffentlichungstag der Patentschrift: **16.03.88**

(51) Int. Cl.⁴: **C 01 B 21/14**, C 07 C 51/42

(21) Anmeldenummer: **84115036.0**

(22) Anmeldetag: **10.12.84**

(54) **Stabilisierte Lösungen von Hydroxylamin oder dessen Salzen in Wasser oder Alkoholen sowie deren Herstellung.**

(30) Priorität: **17.12.83 DE 3345734**

(43) Veröffentlichungstag der Anmeldung: **10.07.85 Patentblatt 85/28**

(45) Bekanntmachung des Hinweises auf die Patenterteilung: **16.03.88 Patentblatt 88/11**

(84) Benannte Vertragsstaaten: **BE CH DE FR GB IT LI NL**

(56) Entgegenhaltungen:
**CHEMICAL ABSTRACTS, Band 97, Nr. 18, 1. November 1982, Seite 137, Nr. 147062h, Columbus, Ohio, US; & JP - A - 82 100 908 (NISSAN CHEMICAL INDUSTRIES LTD.) 23.06.1982**
**CHEMICAL ABSTRACTS, Band 99, Nr. 24, 12. Dezember 1983, Seite 108, Nr. 196962b, Columbus, Ohio, US; & JP - A - 58 69 844 (NISSHIN KAKO CO., LTD.) 26.04.1983**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Grosskinsky, Otto- Alfred, Dr. Chemiker, Semmelweisstrasse 8, D-6700 Ludwigshafen (DE)**
Erfinder: **Frommer, Elmar, Dr. Chemiker, Lisztstrasse 117, D-6700 Ludwigshafen (DE)**
Erfinder: **Ritz, Josef, Dr. Chemiker, Osloer Weg 8, D-6700 Ludwigshafen (DE)**
Erfinder: **Thomas, Erwin, Chemotechniker, Borngasse 12, D-6713 Freinsheim (DE)**
Erfinder: **Weiss, Franz- Josef, Dr. Chemiker, Schilfweg 1, D-6708 Neuhofen (DE)**

**EP 0 147 706 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Lösungen von Hydroxylammoniumsalzen zersetzen sich langsam bei Raumtemperatur und schneller bei erhöhter Temperatur. Dies gilt in vermehrtem Maße für Lösungen von freiem Hydroxylamin. Es hat nicht an Versuchen gefehlt, Lösungen von Hydroxylamin und dessen Salzen zu stabilisieren um eine verbesserte Lagerfähigkeit zu erzielen. So verwendet man als Stabilisierungsmittel Harnstoffderivate entsprechend der US-PS 3 544 270. Aus der US-A- 3 480 391 ist bekannt, daß sich Amidoxime als Stabilisatoren eignen. Entsprechend der US-A- 3 480 392 werden hierfür Hydroxamsäuren empfohlen. Aus der US-A- 3 145 082 ist auch schon bekannt, chelatbildende Mittel, wie das Natriumsalz der Ethylendiamintetraessigsäure als Stabilisierungsmittel zu verwenden. Ferner ist aus JP-A- 82100908 bekannt, daß sich 8-Hydroxychinolin als Stabilisierungsmittel für Hydroxylamin eignet. Die bislang verwendeten Stabilisierungsmittel sind noch verbesserungsbedürftig.

Es war die technische Aufgabe gestellt, stabilisierte Lösungen von Hydroxylamin oder dessen Salzen zur Verfügung zu stellen, die über einen längeren Zeitraum stabil sind und bei denen insbesondere die Zersetzung von freiem Hydroxylamin auch bei schwach erhöhter Temperatur minimiert wird.

Diese Aufgabe wird gelöst durch stabilisierte Lösungen von Hydroxylamin oder dessen Salzen in Wasser oder Alkoholen, gekennzeichnet durch einen Gehalt an 8-Hydroxychinaldinen.

Ferner ist ein Gegenstand der Erfindung ein Verfahren zur Herstellung von stabilisierten Lösungen von Hydroxylamin oder dessen Salzen durch Zugabe von Stabilisatoren, dadurch gekennzeichnet, daß man aus der zu stabilisierenden Lösung den darin gelöst enthaltenen molekularen Sauerstoff durch Behandeln mit Stickstoff, der frei von molekularem Sauerstoff ist, entfernt und dann 8-Hydroxychinaldine zusetzt.

Die gemäß der Erfindung stabilisierten Lösungen von Hydroxylamin oder dessen Salzen haben den Vorteil, daß sie über längere Zeit als bisher stabil sind und insbesondere die Zersetzung von freiem Hydroxylamin auch bei schwach erhöhter Temperatur auf ein Minimum reduziert wird.

Erfindungsgemäß geht man von Lösungen von Hydroxylamin oder dessen Salzen in Wasser oder Alkoholen z. B. $C_1$-$C_4$-Alkanolen aus. Geeignete Salze des Hydroxylamins sind beispielsweise solche mit starken Mineralsäuren wie Schwefelsäure, Salpetersäure, Chlorwasserstoffsäure oder solche mit Fettsäuren, z. B. Essigsäure oder Propionsäure. Aufgrund der verschiedenen Löslichkeiten liegt Hydroxylamin vorzugsweise gelöst in Wasser oder Alkoholen vor, während dessen Salze vorzugsweise als wäßrige Lösungen vorliegen. Der Gehalt an Hydroxylamin oder dessen Salzen beträgt in der Regel von 10 bis 70 Gew. %. Besonders bevorzugt geht man von Lösungen von Hydroxylamin in Wasser aus. Diese haben in der Regel einen pH-Wert von 8 bis 11.

Als Stabilisatoren verwendet man 8-Hydroxychinaldine. Bevorzugt sind solche der Formel I

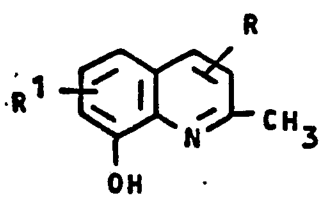

in der R und $R^1$ jeweils ein Wasserstoffatom, eine Hydroxylgruppe, eine $C_1$- bis $C_4$-Alkoxygruppe oder eine $C_1$- bis $C_4$-Alkylgruppe bezeichnen. Geeignete 8-Hydroxychinaldine sind z. B. 6-Methyl-8-hydroxychinaldin, 5, 8-Dihydroxychinaldin. Besonders bevorzugt ist 8-Hydroxychinaldin.

Vorteilhaft verwendet man Chinaldin der Formel I in Mengen von 0,005 bis 1 Gew.-%, insbesondere von 0, 01 bis 0,1 Gew.- %, bezogen auf die zu stabilisierende Lösung an. Es hat sich ferner bewährt, wenn man zusätzlich Polyhydroxybenzole, insbesondere Pyrogallol mitverwendet. Polyhydroxybenzole werden vorteilhaft in Mengen von 0,005 bis 0,1 Gew.-%, bezogen auf die zu stabilisierende Lösung, zugesetzt. Es ist bemerkenswert, daß durch die kombinierte Anwendung von 8-Hydroxychinaldinen und Polyhydroxybenzolen ein synergistischer Effekt erzielt wird.

Stabilisierte Lösungen von Hydroxylamin oder dessen Salzen in Wasser oder Alkoholen werden erfindungsgemäß hergestellt, indem man aus den zu stabilisierenden Lösungen zunächst den darin gelöst enthaltenen molekularen Sauerstoff durch Behandeln mit Stickstoff, der frei von molekularem Sauerstoff ist, verdrängt. Dies erzielt man beispielsweise dadurch, daß man durch die zu stabilisierende Lösung sauerstofffreien Stickstoff leitet, z. B. für 5 bis 10 Minuten. Der angewandte Stickstoff enthält vorteilhaft weniger als 2 ppm molekularen Sauerstoff. Dann gibt man 8-Hydroxychinal dine und gegebenenfalls Polyhydroxybenzole zu und löst diese in der zu stabilisierenden Lösung auf. Hierbei hält man vorteilhaft eine Temperatur von 5 bis 40°C ein. Es ist auch möglich, die Stabilisatoren bereits in gelöstem Zustand, z. B. gelöst in $C_1$-$C_4$-Alkanolen, den zu stabilisierenden Lösungen zuzugeben.

Vorzugsweise vermeidet man eine Kontamination der zu stabilisierenden Lösungen mit Schwermetallen, insbesondere Kupfer oder Edelmetallen, da diese die Zersetzung von Hydroxylamin katalysieren. Es hat sich bewährt, wenn der Gehalt an Schwermetallen kleiner 1 ppm beträgt. Ferner ist es von Vorteil, energiereiche Strahlen durch geeignet eingefärbte Glasbehälter auszuschalten. Schließlich hat es sich bewährt, die stabilisierten Lösungen bei Temperaturen von kleiner 40°C, z. B. bei Temperaturen von 5 bis 20°C, zu lagern.

Stabilisierte Lösungen von Hydroxylamin oder dessen Salzen eignen sich zur Herstellung von Oximen. Der erfindungsgemäße Gegenstand sei an folgendem Beispiel veranschaulicht.

**Beispiel**

Durch eine wäßrige Lösung von Hydroxylamin wird bei 20°C 10 Minuten sauerstoff-freier Stickstoff geleitet und dann der Stabilisator zugefügt. Die Konzentration an Hydroxylamin, die zugegebene Menge und Art des Stabilisators sowie die in Abhängigkeit von der Zeit erzielten Ergebnisse sind aus folgender Tabelle zu entnehmen.

**Tabelle**

| Stabilisator | °C | | | | |
|---|---|---|---|---|---|
| 50 ppm 8-Hydroxy- | | 0 | 743 | 1198 | Stunden |
| chinaldin | 5 | 111,90 | 111,80 | 111,71 | g/l $NH_2OH$ |
| " | 20 | 0 | 404 | 1195 | Stunden |
| | | 111,38 | 111,21 | 111,05 | g/l $NH_2OH$ |
| " | 40 | 0 | 359 | 1194 | Stunden |
| | | 111,51 | 110,62 | 109,57 | g/l $NH_2OH$ |

**Patentansprüche**

1. Stabilisierte Lösungen von Hydroxylamin oder dessen Salzen in Wasser oder Alkoholen, gekennzeichnet durch einen Gehalt an 8-Hydroxychinal dinen.

2. Stabilisierte Lösungen nach Anspruch 1, gekennzeichnet durch einen Gehalt an 8-Hydroxychinaldinen der Formel I

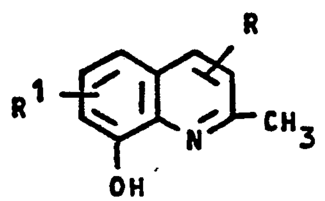

in der R und $R^1$ jeweils ein Wasserstoffatom, eine Hydroxylgruppe, eine $C_1$- bis $C_4$ -Alkoxygruppe oder eine $C_1$-bis $C_4$-Alkylgruppe bezeichnen.

3. Stabilisierte Lösungen nach den Ansprüchen 1 und 2, gekennzeichnet durch einen Gehalt von 0,005 bis 1 Gew.-% Chinaldin der Formel I, bezogen auf die zu stabilisierende Lösung.

4. Stabilisierte Lösungen nach den Ansprüchen 1 bis 3, dadurch gekennzeichnet, daß man von einer wäßrigen Lösung mit einem Gehalt von 10 bis 70 Gew.-% an Hydroxylamin ausgeht.

5. Stabilisierte Lösungen nach den Ansprüchen 1 bis 4, gekennzeichnet durch einen zusätzlichen Gehalt an Pyrogallol.

6. Verfahren zur Herstellung von stabilisierten Lösungen von Hydroxylamin oder dessen Salzen in Wasser oder Alkoholen durch Zusatz von Stabilisatoren, dadurch gekennzeichnet, daß man aus den zu stabilisierenden Lösungen den darin gelöst enthaltenen molekularen Sauerstoff durch Behandeln mit Stickstoff, der frei von molekularem Sauerstoff ist, entfernt und dann 8-Hydroxychinaldine zusetzt.

**Claims**

1. A stabilized solution of hydroxylamine or its salts in water or an alcohol, which contains an 8-hydroxyquinaldine.

2. A stabilized solution as claimed in claim 1, which contains an 8-hydroxyquinaldine of the formula I

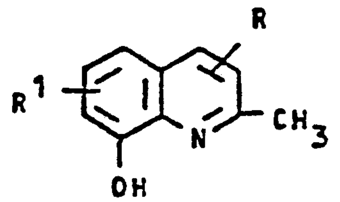

I,

where R and $R^1$ are each hydrogen, hydroxyl, $C_1$-$C_4$alkoxy or $C_1$-$C_4$-alkyl.

3. A stabilized solution as claimed in claims 1 and 2, which contains from 0.005 to 1 % by weight, based on the solution to be stabilized, of a quinaldine of the formula I.

4. A stabilized solution as claimed in claims 1 to 3, wherein the starting solution used is an aqueous solution containing from 10 to 70 % by weight of hydroxylamine.

5. A stabilized solution as claimed in claims 1 to 4, which additionally contains pyrogallol.

6. A process for the preparation of a stabilized solution of hydroxylamine or its salts in water or an alcohol, wherein the molecular oxygen dissolved in the solution to be stabilized is removed from this solution by treatment with nitrogen which is free of molecular oxygen, and an 8-hydroxyquinaldine is then added.

**Revendications**

1. Solutions aqueuses ou alcooliques de l'hydroxylamine ou de ses sels stabilisées, caractérisées en ce qu'elles contiennent des 8-hydroxy-quinaldines.

2. Solutions stabilisées suivant la revendication 1, caractérisées en ce qu'elles contiennent des 8-hydroxy-quinaldines de la formule I

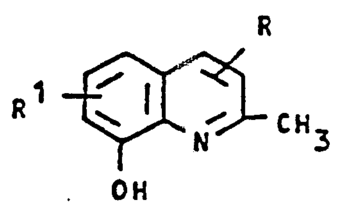

dans laquelle R et $R^1$ peuvent désigner chacun un atome d'hydrogène, un radical oxhydryle, un radical alcoxy en $C_1$ à $C_4$ ou un radical alkyle en $C_1$ à $C_4$.

3. Solutions stabilisées suivant les revendications 1 et 2, caractérisées en ce qu'elles contiennent entre 0,005 et 1 % de leur poids d'une quinaldine de la formule I.

4. Solutions stabilisées suivant les revendications 1 à 3, caractérisées en ce qu'elles sont préparées au départ d'une solution aqueuse d'une teneur en hydroxylamine entre 10 et 70 % en poids.

5. Solutions stabilisées suivant les revendications 1 à 4, caractérisées en ce qu'elles contiennent en outre du pyrogallol.

6. Procédé de préparation de solutions aqueuses ou alcooliques de l'hydroxylamine ou de ses sels stabilisées par l'addition de stabilisants, caractérisé en ce que l'on élimine des solutions à stabiliser l'oxygène moléculaire qui y est dissous par un traitement à l'azote exempt d'oxygène moléculaire, puis on y ajoute des 8-hydroxy-quinaldines.